# FASCICULE DE BREVET EUROPEEN

(11) **EP 3 556 417 B1**
(45) Date de publication et mention de la délivrance du brevet: **06.01.2021**
(21) Numéro de dépôt: 19166853.2
(22) Date de dépôt: 02.04.2019
(51) Int. Cl.: A61M 16/01, A61M 16/10, A61M 16/12

(54) **PROCÉDÉ ET SYSTÈME DE FOURNITURE D'UN MÉLANGE GAZEUX DE PROTOXYDE D'AZOTE ET D'OXYGÈNE**
VERFAHREN UND SYSTEM ZUM LIEFERN EINES GASGEMISCHS AUS DISTICKSTOFFOXID UND SAUERSTOFF
METHOD AND SYSTEM FOR PROVIDING A GAS MIXTURE OF NITROGEN PROTOXIDE AND OXYGEN

(30) Priorité: 20.04.2018 FR 1853491
(43) Date de publication de la demande: 23.10.2019
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: CORNILLAC, Mathieu, 94250 Gentilly (FR); LECOURT, Laurent, 94250 Gentilly (FR)
(74) Mandataire: Air Liquide

(56) Documents cités:
- WO-A2-2016/027097
- GB-A- 191 409 600
- US-A- 4 526 188
- US-A1- 2011 259 325
- US-A1- 2012 318 263

## Description

L'invention porte sur un procédé de fourniture d'un mélange gazeux contenant du protoxyde d'azote (N₂O) et de l'oxygène (O₂), et un système de fourniture d'un mélange gazeux à base de protoxyde d'azote et d'oxygène.

Le mélange gazeux équimolaire (50%/50% molaire) de protoxyde d'azote et d'oxygène a montré une efficacité dans le traitement des douleurs aigües ou chroniques de différentes étiologies, lorsqu'il est administré à un individu, i.e. un patient, par inhalation au moyen d'une interface respiratoire, tel un masque respiratoire, des canules nasales ou analogue.

On a cependant remarqué une forte variabilité d'un patient à un autre concernant l'efficacité, la toxicité et/ou la tolérance de ce mélange gazeux équimolaire (i.e., 50% N₂O/ 50% O₂).

Ainsi, certains patients tolèrent mal ce mélange équimolaire du fait de la teneur de 50% molaire en protoxyde d'azote, et par ailleurs d'autres patients traités avec cette même teneur de 50 % en N₂O pourraient être soulagés avec des teneurs moins élevées. Pour ces patients, il est important de pouvoir réduire la teneur en protoxyde d'azote pour obtenir le meilleur compromis "bénéfice risque".

Ainsi, EP-A-2851076 et EP-A-2851077 ont proposé d'utiliser une teneur de protoxyde d'azote à une teneur comprise entre 15 et 45% pour traiter des douleurs de différentes origines.

Toutefois, en pratique, la réalisation de ce type de mélange gazeux contenant moins de 50% de protoxyde d'azote n'est pas aisée. En effet, une façon simple pour obtenir un tel mélange gazeux serait d'utiliser un mélangeur de gaz classique alimenté en protoxyde d'azote, d'une part, et en oxygène, d'autre part, de manière à réaliser le mélange gazeux N₂O/O₂ à la teneur en protoxyde d'azote désirée et contenant en outre une teneur non-hypoxique d'oxygène, c'est-à-dire typiquement plus de 21% d'oxygène. Ce type de mélangeur de gaz est notamment utilisé en anesthésie comme décrit par US-A-4,526,188.

Par ailleurs, US-A-2012/0318263 enseigne un système vaporiseur d'anesthésie comprenant un mélangeur de gaz alimenté par trois réservoirs de gaz contenant de l'oxygène, du N₂O et de l'air.

Or, pour des raisons de sécurité (e.g. risque de surdosage en N₂O) et de réglementation, il est souvent difficile, parfois même impossible, d'utiliser ce type de mélangeur classique pour réaliser des mélanges N₂O/O₂ destinés à être administrés à des patients, en particulier lorsque le patient est traité à son domicile, donc sans opérateur présent sur place pour réaliser le mélange gazeux souhaité.

En effet, un mélangeur classique permet d'obtenir des teneurs en N₂O inférieures à 50 % mais la teneur en oxygène augmente alors et devient supérieure à 50%. Or, l'oxygène est un médicament et dès lors augmenter la teneur en oxygène n'est pas anodin, notamment du fait de la toxicité de l'oxygène à forte FiO₂.

Par ailleurs, le fait de devoir utiliser deux bouteilles de gaz n'est pas pratique car posant des problèmes de logistique, d'approvisionnement, d'encombrement, de sécurité, de maintenance...

En outre, ces mélangeurs classiques sont souvent volumineux et encombrants, donc, là encore, peu compatibles avec un usage au domicile des patients ou en service de ville, c'est-à-dire chez des praticiens de santé exerçant hors de l'hôpital, par exemple en cabinet de kinésithérapie ou cabinet dentaire ...

Au vu de cela, le problème qui se pose est de pouvoir réaliser facilement des mélanges gazeux à base de protoxyde d'azote et d'oxygène, en particulier ayant une teneur en protoxyde d'azote inférieure à 50% (% molaire), sans rencontrer les problèmes susmentionnés, en particulier de sécurité (e.g., surdosage), d'encombrement et de teneur en oxygène trop élevée.

La solution selon la présente invention concerne alors un procédé de fourniture d'un mélange gazeux contenant du protoxyde d'azote (N₂O) et de l'oxygène (O₂) comprenant les étapes de :
a) fournir un prémélange de N₂O et de O₂ contenant une teneur initiale en N₂O comprise entre 40% et 60% molaire, le reste étant de l'oxygène, ledit prémélange de N₂O et de O₂ provenant d'un récipient de gaz,
b) diluer le prémélange de N₂O et de O₂ avec de l'air délivré par une micro-soufflante, aussi appelée turbine ou compresseur, et
c) récupérer un mélange final contenant une teneur finale en N₂O inférieure à la teneur initiale de N₂O dans le prémélange de N₂O et de O₂.

Selon le cas, le procédé de l'invention peut comprendre l'une ou plusieurs des caractéristiques techniques suivantes :
- le prémélange de N₂O et O₂ contient une teneur initiale en N₂O comprise entre 45% et 55% molaire, le reste étant de l'oxygène.
- le prémélange de N₂O et de O₂ contient 50% molaire de N₂O et 50% molaire d'oxygène, c'est-à-dire qu'il s'agit d'un mélange équimolaire de N₂O et de O₂.
- le prémélange de N₂O et de O₂ est délivré par une bouteille de gaz.
- le prémélange de N₂O et de O₂ est stocké dans une bouteille de gaz à une pression comprise entre 1 et 300 bar abs.
- le prémélange de N₂O et de O₂ est délivré par un récipient de gaz équipé d'un bloc robinet ou d'un bloc robinet à détendeur de gaz intégré (RDI).
- le prémélange de N₂O et de O₂ est délivré par une bouteille de gaz équipée d'un bloc robinet ou d'un bloc robinet à détendeur de gaz intégré (RDI) protégé par un capotage de protection, encore appelé « chapeau ».
- la micro-soufflante est du type à vitesse rapide, par exemple pouvant fonctionner à une vitesse allant jusqu'à 70 000 tours/min, typiquement moins de 40 000 tr/min.
- à l'étape b), la dilution du prémélange de N₂O et de O₂ avec de l'air est opéré dans un mélangeur de gaz comprenant une chambre de mélange, en particulier un réservoir sous pression.
- à l'étape c), on récupère un mélange final contenant une teneur finale en N₂O comprise entre 15 et 50% molaire.
- à l'étape c), on récupère un mélange final contenant au moins N₂O, O₂ et N₂.
- une étape additionnelle de contrôle de la teneur en oxygène (FiO₂) dans le prémélange gazeux de N₂O et de O₂ et/ou dans le mélange gazeux final.
- à l'étape c), on récupère un mélange final à un débit final (Q_{final}) inférieur ou égal à 100 L/min, typiquement entre 2 et 30 L/min.
- à l'étape c), on récupère un mélange final à une pression de sortie inférieure ou égale à 5 bar abs.
- une autre étape additionnelle de déclenchement d'une alarme sonore ou visuelle lorsque la teneur en O₂ mesurée ne correspond pas à la teneur en oxygène attendue ou désirée, par exemple du fait d'une erreur de source de prémélange (i.e. mauvaise bouteille de gaz), d'un problème de stockage induisant une variation de teneur en oxygène dans le prémélange, d'un défaut de qualité de produit....

L'invention porte par ailleurs sur un système de fourniture de gaz comprenant :
- une source de prémélange N₂O/O₂ contenant une teneur initiale en N₂O comprise entre 40% et 60% molaire, le reste étant de l'oxygène, la source de prémélange N₂O/O₂ étant communication fluidique avec une chambre de mélange de gaz par l'intermédiaire d'une ligne d'acheminement de prémélange N₂O/O₂, la source de prémélange N₂O/O₂ étant un récipient de gaz,
- une source d'air en communication fluidique avec la chambre de mélange de gaz par l'intermédiaire d'une ligne d'acheminement d'air, la source d'air étant une micro-soufflante, encore appelée turbine ou compresseur,
- une chambre de mélange de gaz pour réaliser un mélange final de gaz contenant une teneur finale en N₂O à partir du prémélange N₂O amené par la ligne d'acheminement de prémélange N₂O/O₂ et d'air amené par la ligne d'acheminement d'air,
- un dispositif de contrôle de débit d'air agencé sur la ligne d'acheminement d'air pour contrôler le débit d'air au sein de la ligne d'acheminement d'air,
- un dispositif de contrôle de débit de prémélange N₂O/O₂ agencé sur la ligne d'acheminement de prémélange N₂O/O₂ pour contrôler le débit de prémélange N₂O/O₂ au sein de la ligne d'acheminement de prémélange N₂O/O₂,
- une interface homme-machine (IHM) permettant à un opérateur de saisir plusieurs paramètres comprenant la teneur initiale en N₂O dans le prémélange N₂O/O₂, la teneur en N₂O dans le mélange final et un débit final de gaz (Q_{final}) désiré du mélange final réalisé dans la chambre de mélange de gaz, et
- des moyens de calcul configurés pour agir sur le dispositif de contrôle de débit d'air et/ou sur le dispositif de contrôle de débit de prémélange N₂O/O₂ de manière à ajuster le débit d'air au sein de la ligne d'acheminement d'air et/ou le débit de prémélange N₂O/O₂ en fonction des paramètres saisis par l'opérateur sur l'interface homme-machine,
et dans lequel ladite interface homme-machine est reliée électriquement auxdits moyens de calcul.

Selon le cas, le système de fourniture de gaz de l'invention peut comprendre l'une ou plusieurs des caractéristiques techniques suivantes :
- la source de prémélange N₂O/O₂ est une bouteille de gaz.
- la source de prémélange N₂O/O₂ est une bouteille de gaz équipée d'un bloc robinet ou d'un bloc robinet à détendeur de gaz intégré (RDI).
- la bouteille de gaz est équipée d'un bloc robinet à détendeur de gaz intégré (RDI) avec une prise ou raccord de sortie de gaz à basse pression raccordé fluidiquement avec la ligne d'acheminement de prémélange N₂O/O₂.
- la bouteille de gaz est en métal ou alliage métallique, ou en matériaux composites, par exemple en acier, en alliage d'aluminium ou autre.
- le dispositif de contrôle de débit d'air comprend une vanne, en particulier une vanne à ouverture proportionnelle, en particulier une électrovanne.
- le dispositif de contrôle de débit de prémélange N₂O/O₂ comprend une vanne, en particulier une vanne à ouverture proportionnelle, en particulier une électrovanne.
- la chambre de mélange de gaz est configurée pour réaliser un mélange de N₂O, d'oxygène et d'air.
- la chambre de mélange de gaz comprend un réservoir sous pression, c'est-à-dire dans lequel règne une pression gazeuse supérieure à la pression atmosphérique (i.e. > 1 bar abs).
- il comprend des moyens d'alimentation électrique, telle une prise électrique secteur ou une ou plusieurs batteries ou analogue, notamment une batterie rechargeable.
- les moyens de calcul comprennent une carte électronique à microprocesseur, notamment à microcontrôleur.
- il comprend une coque ou carcasse externe dans laquelle sont agencés la turbine, la chambre de mélange de gaz, les moyens de calcul et le dispositif de contrôle de débit d'air.
- la source de prémélange N₂O/O₂ est reliée fluidiquement à la chambre de mélange de gaz par l'intermédiaire de la ligne d'acheminement de prémélange N₂O/O₂ et d'une conduite de gaz flexible agencée entre la ligne d'acheminement de prémélange et la source de prémélange N₂O/O₂.
- la conduite de gaz flexible est raccordée fluidiquement à la ligne d'acheminement de prémélange via un connecteur ou analogue.
- la source de prémélange N₂O/O₂ est une bouteille de gaz équipée d'un bloc robinet ou d'un bloc robinet à détendeur de gaz intégré (RDI).
- la conduite de gaz flexible est raccordée fluidiquement au bloc robinet ou RDI équipant une bouteille de gaz.
- il comprend en outre une ligne de récupération de gaz en communication fluidique avec la chambre de mélange de gaz pour récupérer au moins une partie du mélange final réalisé dans ladite chambre de mélange de gaz et acheminer le mélange final au débit final (Q_{final}) saisi par l'opérateur sur l'IHM.
- l'IHM comprend un écran tactile, de préférence à affichage en couleurs.
- la ligne de récupération de gaz est en communication fluidique avec une interface respiratoire, en particulier un masque facial ou analogue.
- il comprend en outre un dispositif débitmètre agencé en aval de la chambre de mélange de gaz de manière à mesurer le débit du mélange gazeux provenant de la chambre de mélange.
- il comprend en outre au moins une sonde, c'est-à-dire un (ou des) capteur ou analyseur, permettant d'opérer des mesures de teneur en oxygène agencée(s) en amont et/ou en aval de la chambre de mélange de gaz pour mesurer la teneur en oxygène dans le prémélange N₂O/O₂ et/ou dans le mélange final, en particulier sur la ligne d'acheminement de prémélange N₂O/O₂ et/ou sur la ligne de récupération de gaz.
- au moins une sonde peut être connecté électriquement aux moyens de calcul.
- une alarme sonore et/ou visuelle, est déclenchée en cas de détection d'une teneur anormale en oxygène, en particulier dans le mélange initial, c'est-à-dire le prémélange .
- il comprend des moyens d'alarme sonore ou visuelle connectés électriquement reliés aux moyens de calcul ou à l'analyseur d'oxygène.
- le débit final de gaz (Q_{final}) désiré est inférieur ou égal à 100 L/min.

Dans le cadre de l'invention, les teneurs sont préférentiellement exprimées en « % molaire » ; toutefois, on pourrait aussi les exprimer en « % volumique ».

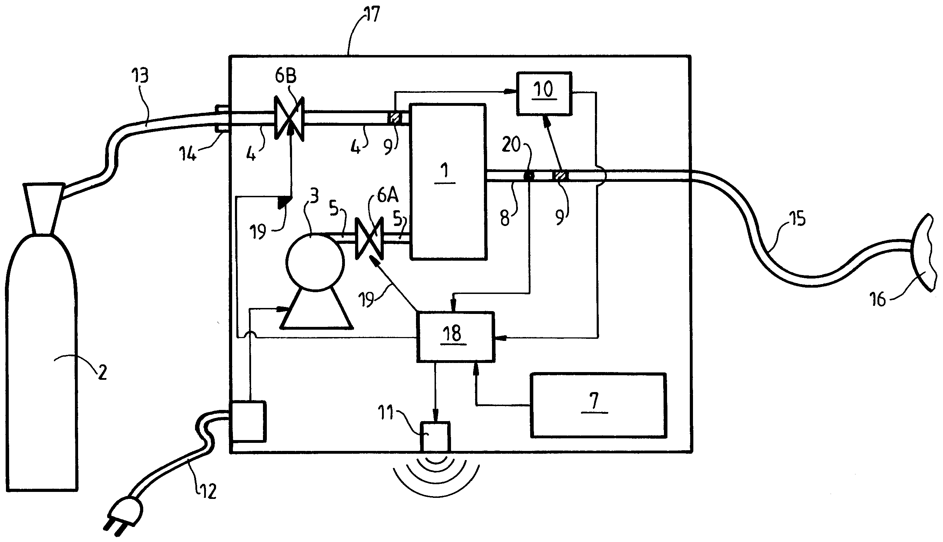

L'invention va maintenant être mieux comprise grâce à la description détaillée suivante, faite à titre illustratif mais non limitatif, en référence à la Figure annexée qui est un schéma de principe d'un système de fourniture de gaz selon la présente invention.

La Figure annexée représente un schéma de principe d'un système de fourniture de gaz d'un mélange gazeux contenant du protoxyde d'azote (N₂O) et de l'oxygène (O₂) selon la présente invention.

Ce système de fourniture de gaz comprend une chambre de mélange de gaz 1, tel un réservoir sous pression, en communication fluidique avec une source 2 de prémélange N₂O/O₂ contenant une teneur initiale en N₂O comprise entre 40% et 60% molaire, le reste étant de l'oxygène, par exemple une bouteille de gaz contenant un mélange binaire N₂O/O₂ équimolaire (50%/50%).

La source 2 de prémélange N₂O/O₂ est reliée fluidiquement à la chambre de mélange de gaz 1 par l'intermédiaire d'une ligne d'acheminement 4 de prémélange N₂O/O₂, tel un conduit ou passage de gaz ou analogue, de manière alimenter ladite chambre de mélange de gaz 1 avec le prémélange gazeux N₂O/O₂. La source 2 de prémélange N₂O/O₂ peut être reliée directement à la ligne d'acheminement 4 ou indirectement, c'est-à-dire par l'intermédiaire d'une canalisation flexible 13 par exemple ou autre.

Par ailleurs, une source d'air 3 qui est une turbine, i.e. micro-soufflante, est également en communication fluidique avec la chambre de mélange de gaz 1 par l'intermédiaire d'une ligne d'acheminement d'air 5, tel un conduit ou passage de gaz ou analogue, de manière à alimenter ladite chambre de mélange de gaz 1 avec de l'air délivré par la micro-soufflante.

Des moyens d'alimentation électrique 12, telle une prise de raccordement au secteur ou une (ou plusieurs) batterie, alimentent la source d'air 3, i.e. micro-soufflante, en courant électrique, et d'autres éléments du système, en particulier des moyens de calcul 18, telle une carte électronique à microprocesseur.

Afin de contrôler le débit d'air au sein de la ligne d'acheminement d'air 5, on prévoit un dispositif 6A de contrôle de débit d'air agencé sur la ligne d'acheminement d'air 5. De manière analogue, un dispositif 6B de contrôle de débit de prémélange N₂O/O₂ est agencé sur la ligne d'acheminement de prémélange N₂O/O₂ 4.

Ces dispositifs de contrôle 6A, 6B sont préférentiellement des électrovannes proportionnelles permettant d'obturer plus ou moins le passage de gaz dans les lignes 4, 5 d'acheminement d'air et de prémélange N₂O/O₂ en fonction des débits d'air et de prémélange N₂O/O₂ désirés, et ainsi contrôler les débits de gaz alimentant la chambre 1 de mélange. Ces dispositifs de contrôle 6A, 6B sont contrôlés 19 par les moyens de calcul 18, aussi appelés moyens de pilotage.

Tout ou partie des composants du système, en particulier la source d'air 3, les dispositifs de contrôle 6A, 6B, les moyens de calcul 18 et la chambre 1, sont agencés au sein d'un boitier 17 formant carcasse externe.

Par ailleurs, il est aussi prévu une IHM 7, c'est-à-dire une interface homme-machine, permettant à un opérateur, par exemple du personnel soignant, voire le patient lui-même, de saisir différents paramètres, en particulier la teneur initiale en N₂O dans le prémélange N₂O/O₂, la teneur en N₂O dans le mélange final et un débit final de gaz (Q_{final}) désiré du mélange gazeux final réalisé dans la chambre de mélange de gaz 1. L'IHM 7 comprend préférentiellement un écran tactile, avantageusement avec affichage en couleurs.

La chambre de mélange de gaz 1 est alimentée avec de l'air et du prémélange N₂O/O₂ à des débits appropriés, permettant d'obtenir la teneur en N₂O finale désirée au sein de la chambre de mélange de gaz 1.

En aval de la chambre de mélange de gaz 1 est agencée une ligne de récupération de gaz 8 en communication fluidique avec la chambre de mélange de gaz 1 au sein de laquelle est récupéré au moins une partie du mélange gazeux réalisé dans la chambre de mélange de gaz 1. La ligne de récupération de gaz 8 en communication fluidique avec un conduit flexible 15 acheminant le mélange gazeux provenant de la chambre 1 et de la ligne 8 jusqu'à une interface respiratoire 16, tel un masque respiratoire, par exemple un masque facial, délivrant le gaz au patient à traiter. Préférentiellement, le gaz sera humidifié et/ou chauffé avant sa fourniture au patient.

Le système de l'invention couple donc un réservoir-mélangeur 1, une source de prémélange N₂O/O₂ typiquement équimolaire (50%/50%), telle une bouteille de gaz, et un dispositif de génération d'air ou source d'air 3, à savoir une turbine ou analogue. Ce système permet, à partir de tels mélanges préformés de protoxyde d'azote et d'oxygène (50%/50% mol.) et de l'air généré par la source d'air 3 d'obtenir un mélange moins riche en protoxyde d'azote et dont la teneur en oxygène reste non-hypoxique, malgré la dilution opérée par l'ajout de l'air au prémélange.

Le système de l'invention permet de saisir, via l'IHM 7, la teneur en N₂O désirée, c'est-à-dire celle qui est la plus adaptée au traitement d'un patient donné, ainsi que le débit de gaz souhaité en fonction du patient considéré. Les calculs se font alors en fonction de ces deux paramètres.

Ainsi, en fonction de la prescription médicale et des besoins du patient, la sélection de la teneur finale en N₂O désirée se fait au niveau de l'IHM 7 par exemple en choisissant une teneur en protoxyde d'azote donnée comprise entre 20 % et 50% (en vol ou en mol). Après saisie au niveau de l'IHM 7 d'une teneur en N₂O désirée et du débit souhaité, les moyens de calcul 18 commandent le dispositif de contrôle de débit d'air 6A et/ou le dispositif de contrôle de débit 6B de prémélange N₂O/O₂ de sorte qu'un ajustement du/des débit(s) d'air et/ou de prémélange N₂O/O₂ soit opéré au sein d'une ou des lignes d'acheminement 5, 4, par exemple une ouverture plus ou moins grande des vannes proportionnelles.

A titre d'exemple, l'IHM 7 peut proposer plusieurs choix de mélanges préenregistrés, par exemple:
- choix « 0 » correspondant à une teneur finale de 50% en N₂O (pas de dilution avec de l'air),
- choix « 1 » correspondant à une teneur finale de 40% en N₂O (i.e. légère dilution avec de l'air),
- choix « 2 » correspondant à une teneur finale de 30% en N₂O (i.e. dilution moyenne avec de l'air), et
- choix « 3 » correspondant à une teneur finale de 20% en N₂O (i.e. forte dilution avec de l'air).

Ces différents choix 0 à 3 permettent chez un même patient, une régulation ou un ajustage des doses de N₂O administrées en fonction des événements indésirables ressentis par le patient.

L'utilisateur ou le personnel soignant sélectionne ou saisit par ailleurs le débit de gaz en sortie du mélangeur 1 (i.e. mélange N₂O/O₂/air) qui est le plus adapté aux besoins du patient, typiquement un débit adapté au volume minute du patient en prenant en compte l'interface respiratoire utilisée pour assurer la délivrance du gaz au patient, par exemple un masque facial. Par exemple, le débit de gaz de sortie (i.e. mélange N₂O/O₂/air) peut être compris entre 6 et 20 L/min, par exemple inférieur ou égal à 15 L/min.

L'IHM peut aussi intégrer l'ensemble des pourcentages de protoxyde d'azote compris entre 50% et 15%. L'opérateur choisit alors le pourcentage de 15% à 50 % en fonction du mélange voulu.

Le Tableau suivant donne des exemples de valeurs de débits applicables correspondant à ces 4 choix pour un mélange équimolaire de N₂O/O₂ (i.e. 50%/50%).

**Tableau**

| Choix n° | 0 | 1 | 2 | 3 |
|---|---|---|---|---|
| Teneur finale en N₂O désirée (% molaire) | 50% | 40% | 30% | 20% |
| Débit prémélange N₂O/O₂ (L/min) | 15 | 12 | 9 | 6 |
| Débit d'air (L/min) | 0 | 3 | 6 | 9 |
| Débit total désiré (L/min) | 15 | 15 | 15 | 15 |

Apres connexion fluidique de la bouteille 2 au système, on peut prévoir de vérifier la teneur en O₂ du prémélange N₂O/O₂ pour s'assurer d'une absence de démélange. Pour ce faire, on peut agencer un capteur d'oxygène 9 sur la ligne 4 d'acheminement de prémélange N₂O/O₂, en amont du réservoir de mélange 1, lequel peut être relié à un analyseur de FiO₂ 10 ou analogue, lui-même relié aux moyens de calcul 18 non pas forcément pour ce premier capteur. Ainsi, lorsqu'une teneur en oxygène inappropriée est détectée, par exemple une teneur correspondant à un démélange, c'est-à-dire lorsque la teneur en oxygène dans le gaz n'est pas conforme à la valeur attendue, une alarme 11 sonore et/ou visuelle peut être déclenchée par les moyens de calcul 18 de manière à en informer l'utilisateur, voire même verrouiller le système par sécurité par exemple en agissant sur une ou les électrovannes des dispositifs de contrôle 6A, 6B.

De même, on peut prévoir d'incorporer un autre capteur d'oxygène 9 dans le système, par exemple en aval de la chambre de mélange de gaz 1, en particulier sur la ligne de récupération de gaz 8 qui est en communication fluidique avec ladite chambre de mélange de gaz 1. Cette autre mesure de teneur en oxygène (FiO₂) permet de s'assurer que le mélange gazeux obtenu dans la chambre de mélange de gaz 1 est conforme aux réglages pour ce qui concerne les teneurs en oxygène et en protoxyde d'azote. Là encore, une alarme 11 sonore et/ou visuelle peut être déclenchée en cas de détection d'une teneur non-conforme qui serait le reflet d'un débit différent de celui souhaité et réglé.

De façon analogue, on peut prévoir d'insérer un dispositif débitmètre 20 en aval de la chambre 1 du système de l'invention de manière à s'assurer que le débit final (Q_{final}) réel est conforme au débit de consigne fixé au niveau de l'IHM 7. Ce dispositif débitmètre 20 est lui aussi relié électriquement aux moyens de calcul 18 de manière à détecter toute valeur de débit non-conforme et la signaler par le déclenchement d'une alarme 11.

La durée d'administration de gaz doit être conforme à la prescription médicale. Par exemple, dans le cas d'une douleur chronique, il est recommandé d'opérer une administration de gaz pendant 1 heure environ. Le temps d'inhalation est préférentiellement affiché sur l'écran de l'IHM. L'arrêt de l'administration est effectué après 1 heure. Préférentiellement, on opère aussi une détermination et un enregistrement de l'observance du patient à son traitement. Bien entendu, d'autres durées peuvent être fixées en fonction du traitement à mettre en œuvre.

On peut prévoir aussi d'autres sécurités, par exemple l'initiation du traitement et le démarrage de la fourniture du mélange au patient peut se faire uniquement après validation d'un code d'identification du patient et/ou du personnel soignant.

De même, on peut prévoir un système de contrôle de dose pour limiter la dose maximale quotidienne délivrée à un même patient et ce, notamment pour éviter les mésusages et/ou un surdosage éventuel.

Les infos sont mémorisées et/ou transmises par exemple au médecin en charge du patient considéré à des fins de validation de l'observance et des doses administrées (e.g., % moyen sur durée d'une heure, durée dans chaque position 0 à 3 du sélecteur ...). On peut aussi suivre et enregistrés d'autres paramètres physiologiques, comme les teneurs en oxygène mesurées (SpO₂) ou autres.

## Revendications

1. Procédé de fourniture d'un mélange gazeux contenant du protoxyde d'azote (N₂O) et de l'oxygène (O₂) comprenant les étapes de :
a) fournir un prémélange de N₂O et de O₂ contenant une teneur initiale en N₂O comprise entre 40% et 60% molaire, le reste étant de l'oxygène, ledit prémélange de N₂O et de O₂ provenant d'un récipient de gaz,
b) diluer le prémélange de N₂O et de O₂ avec de l'air délivré par une micro-soufflante, et
c) récupérer un mélange final contenant une teneur finale en N₂O inférieure à la teneur initiale de N₂O dans le prémélange de N₂O et de O₂.

2. Procédé selon la revendication 1, **caractérisé en ce que** le prémélange de N₂O et de O₂ contient une teneur initiale en N₂O comprise entre 45% et 55% molaire, le reste étant de l'oxygène.

3. Procédé selon l'une des revendications précédentes, **caractérisé en ce que** le prémélange de N₂O et de O₂ contient 50% molaire de N₂O et 50% molaire d'oxygène.

4. Procédé selon l'une des revendications précédentes, **caractérisé en ce que** le prémélange de N₂O et de O₂ est délivré par une bouteille de gaz.

5. Procédé selon la revendication 1, **caractérisé en ce que** le prémélange de N₂O et de O₂ est délivré par un récipient de gaz équipé d'un bloc robinet à détendeur de gaz intégré.

6. Procédé selon la revendication 1, **caractérisé en ce qu'**à l'étape b), la dilution du prémélange de N₂O et de O₂ avec de l'air est opéré dans un mélangeur de gaz comprenant une chambre de mélange, en particulier un réservoir sous pression.

7. Procédé selon la revendication 1, **caractérisé en ce qu'**à l'étape c), on récupère un mélange final contenant une teneur finale en N₂O comprise entre 15 et 50% molaire, de préférence moins de 40% molaire.

8. Procédé selon l'une des revendications 1 ou 7, **caractérisé en ce qu'**à l'étape c), on récupère un mélange final contenant au moins N₂O, O₂ et N₂.

9. Procédé selon la revendication 1, **caractérisé en ce qu'**il comprend en outre une étape additionnelle de contrôle de la teneur en oxygène (FiO₂) dans le prémélange de N₂O et de O₂ et/ou dans le mélange final.

10. Système de fourniture de gaz comprenant :
- une source de prémélange N₂O/O₂ (2) contenant une teneur initiale en N₂O comprise entre 40% et 60% molaire, le reste étant de l'oxygène, la source de prémélange N₂O/O₂ (2) étant communication fluidique avec une chambre de mélange de gaz (1) par l'intermédiaire d'une ligne d'acheminement de prémélange N₂O/O₂ (4), la source de prémélange N₂O/O₂ (2) étant un récipient de gaz,
- une source d'air (3) en communication fluidique avec la chambre de mélange de gaz (1) par l'intermédiaire d'une ligne d'acheminement d'air (5), la source d'air (3) étant une micro-soufflante,
- une chambre de mélange de gaz (1) pour réaliser un mélange final de gaz contenant une teneur finale en N₂O à partir du prémélange N₂O amené par la ligne d'acheminement de prémélange N₂O/O₂ (4) et d'air amené par la ligne d'acheminement d'air (5),
- un dispositif de contrôle de débit d'air (6A) agencé sur la ligne d'acheminement d'air (5) pour contrôler le débit d'air au sein de la ligne d'acheminement d'air (5) et/ou un dispositif de contrôle de débit de prémélange N₂O/O₂ (6B) agencé sur la ligne d'acheminement de prémélange N₂O/O₂ (4) pour contrôler le débit de prémélange N₂O/O₂ au sein de la ligne d'acheminement de prémélange N₂O/O₂ (4),
- une interface homme-machine (7) permettant à un opérateur de saisir plusieurs paramètres comprenant la teneur initiale en N₂O dans le prémélange N₂O/O₂, la teneur en N₂O dans le mélange final et un débit final de gaz (Q_{final}) désiré du mélange final réalisé dans la chambre de mélange de gaz (1), et
- des moyens de calcul (18) configurés pour agir (19) sur le dispositif de contrôle de débit d'air (6A) et/ou sur le dispositif de contrôle de débit de prémélange N₂O/O₂ (6B) de manière à ajuster le débit d'air au sein de la ligne d'acheminement d'air (5) et/ou le débit de prémélange N₂O/O₂ en fonction des paramètres saisis par l'opérateur sur l'interface homme-machine (7),
et dans lequel ladite interface homme-machine (7) est reliée électriquement auxdits moyens de calcul (18).

11. Système de fourniture de gaz selon la revendication 10, **caractérisé en ce que** la source de prémélange N₂O/O₂ (2) est une bouteille de gaz.

12. Système de fourniture de gaz selon la revendication 10, **caractérisé en ce que** le dispositif de contrôle de débit d'air (6A) et le dispositif de contrôle de débit de prémélange N₂O/O₂ (6B) comprennent chacun une électrovanne, en particulier une électrovanne à ouverture proportionnelle.

13. Système de fourniture de gaz selon la revendication 10, **caractérisé en ce que** les moyens de calcul (18) comprennent au moins un microprocesseur, en particulier un microcontrôleur.

14. Système de fourniture de gaz selon la revendication 10, **caractérisé en ce qu'**il comprend en outre une ligne de récupération de gaz (8) en communication fluidique avec la chambre de mélange de gaz (1) pour récupérer au moins une partie du mélange final réalisé dans ladite chambre de mélange de gaz (1) et acheminer le mélange final au débit final (Q_{final}) saisi par l'opérateur sur l'interface homme-machine (7).

15. Système de fourniture de gaz selon la revendication 10, **caractérisé en ce que** l'interface homme-machine (7) comprend un écran tactile, de préférence à affichage en couleurs.

## Patentansprüche

1. Verfahren zum Liefern eines Gasgemisches, das Distickstoffoxid (N₂O) und Sauerstoff (O₂) enthält, umfassend die folgenden Schritte:
a) Liefern eines Vorgemisches aus N₂O und O₂, das einen Anfangsgehalt an N₂O zwischen 40 Mol-% und 60 Mol-% enthält, wobei der Rest Sauerstoff ist, wobei das Vorgemisch aus N₂O und O₂ aus einem Gasbehälter stammt,
b) Verdünnen des Vorgemisches aus N₂O und O₂ mit Luft, die von einem Mikrogebläse abgegeben wird, und
c) Rückgewinnen eines Endgemisches, das einen Endgehalt an N₂O enthält, der niedriger ist als der Anfangsgehalt an N₂O in dem Vorgemisch aus N₂O und O₂.

2. Verfahren nach Anspruch 1, **dadurch gekennzeichnet, dass** das Vorgemisch aus N₂O und O₂ einen Anfangsgehalt an N₂O zwischen 45 Mol-% und 55 Mol-% enthält, wobei der Rest Sauerstoff ist.

3. Verfahren nach einem der vorstehenden Ansprüche, **dadurch gekennzeichnet, dass** das Vorgemisch aus N₂O und O₂ 50 Mol-% N₂O und 50 Mol-% Sauerstoff enthält.

4. Verfahren nach einem der vorstehenden Ansprüche, **dadurch gekennzeichnet, dass** das Vorgemisch aus N₂O und O₂ von einer Gasflasche abgegeben wird.

5. Verfahren nach Anspruch 1, **dadurch gekennzeichnet, dass** das Vorgemisch aus N₂O und O₂ von einem Gasbehälter abgegeben wird, der mit einem Schließhahnblock mit integriertem Gasdruckminderer ausgestattet ist.

6. Verfahren nach Anspruch 1, **dadurch gekennzeichnet, dass** im Schritt b) die Verdünnung des Vorgemisches aus N₂O und O₂ mit Luft in einem Gasmischer durchgeführt wird, der eine Mischkammer, insbesondere einen Druckbehälter umfasst.

7. Verfahren nach Anspruch 1, **dadurch gekennzeichnet, dass** im Schritt c) ein Endgemisch rückgewonnen wird, das einen Endgehalt an N₂O zwischen 15 Mol-% und 50 Mol-%, bevorzugt weniger als 40 Mol-%, enthält.

8. Verfahren nach einem der Ansprüche 1 oder 7, **dadurch gekennzeichnet, dass** im Schritt c) ein Endgemisch rückgewonnen wird, das mindestens N₂O, O₂ und N₂ enthält.

9. Verfahren nach Anspruch 1, **dadurch gekennzeichnet, dass** es weiter einen zusätzlichen Schritt der Steuerung des Sauerstoffgehalts (FiO₂) in dem Vorgemisch aus N₂O und O₂ und/oder in dem Endgemisch umfasst.

10. System zum Liefern von Gas, umfassend:
- eine N₂O/O₂-Vorgemischquelle (2), die einen Anfangsgehalt an N₂O zwischen 40 Mol-% und 60 Mol-% enthält, wobei der Rest Sauerstoff ist, wobei die N₂O/O₂-Vorgemischquelle (2) mittels einer N₂O/O₂-Vorgemischförderleitung (4) in Fluidkommunikation mit einer Gasmischkammer (1) steht, wobei die N₂O/O₂-Vorgemischquelle (2) ein Gasbehälter ist,
- eine Luftquelle (3) in Fluidkommunikation mit der Gasmischkammer (1) mittels einer Luftförderleitung (5), wobei die Luftquelle (3) ein Mikrogebläse ist,
- eine Gasmischkammer (1) zum Erzeugen eines Gasendgemisches, das einen Endgehalt an N₂O enthält, aus dem durch die N₂O/O₂-Vorgemischförderleitung (4) zugeführten N₂O-Vorgemisch und der durch die Luftförderleitung (5) zugeführten Luft,
- eine an der Luftförderleitung (5) angeordnete Luftdurchsatz-Steuerungsvorrichtung (6A) zur Steuerung des Luftdurchsatzes innerhalb der Luftförderleitung (5) und/oder eine an der N₂O/O₂-Vorgemischförderleitung (4) angeordnete N₂O/O₂-Vorgemischdurchsatz-Steuerungsvorrichtung (6B) zur Steuerung des N₂O/O₂-Vorgemischdurchsatzes innerhalb der N₂O/O₂-Vorgemischförderleitung (4),
- eine Mensch-Maschine-Schnittstelle (7), die es einem Bediener ermöglicht, mehrere Parameter einzugeben, die den Anfangsgehalt an N₂O in dem N₂O/O₂-Vorgemisch, den Gehalt an N₂O in dem Endgemisch und einen gewünschten Gasenddurchsatz (Q_{final}) des in der Gasmischkammer (1) erzeugten Endgemisches umfassen, und
- Berechnungsmittel (18), die konfiguriert sind, um auf die Luftdurchsatz-Steuerungsvorrichtung (6A) und/oder auf die N₂O/O₂-Vorgemischdurchsatz-Steuerungsvorrichtung (6B) so zu wirken (19), dass der Luftdurchsatz in der Luftförderleitung (5) und/oder der N₂O/O₂-Vormischdurchsatz in Abhängigkeit von den vom Bediener an der Mensch-Maschine-Schnittstelle (7) eingegebenen Parametern eingestellt wird,
und wobei die Mensch-Maschine-Schnittstelle (7) elektrisch mit den Berechnungsmitteln (18) verbunden ist.

11. System zum Liefern von Gas nach Anspruch 10, **dadurch gekennzeichnet, dass** die N₂O/O₂-Vorgemischquelle (2) eine Gasflasche ist.

12. System zum Liefern von Gas nach Anspruch 10, **dadurch gekennzeichnet, dass** die Luftdurchsatz-Steuerungsvorrichtung (6A) und die N₂O/O₂-Vorgemischdurchsatz-Steuerungsvorrichtung (6B) jeweils ein Elektroventil, insbesondere ein Elektroventil mit proportionaler Öffnung, umfassen.

13. System zum Liefern von Gas nach Anspruch 10, **dadurch gekennzeichnet, dass** die Berechnungsmittel (18) mindestens einen Mikroprozessor, insbesondere einen Mikrocontroller, umfassen.

14. System zum Liefern von Gas nach Anspruch 10, **dadurch gekennzeichnet, dass** es weiter eine Gasrückgewinnungsleitung (8) in Fluidkommunikation mit der Gasmischkammer (1) umfasst, um zumindest einen Teil des in der Gasmischkammer (1) erzeugten Endgemisches rückzugewinnen und das Endgemisch an den Enddurchsatz (Q_{final}) zu fördern, der von dem Bediener an der Mensch-Maschine-Schnittstelle (7) eingegeben wird.

15. System zum Liefern von Gas nach Anspruch 10, **dadurch gekennzeichnet, dass** die Mensch-Maschine-Schnittstelle (7) einen Touchscreen, bevorzugt mit einer Farbanzeige, umfasst.

## Claims

1. Method for providing a gaseous mixture containing nitrogen protoxide (N₂O) and oxygen (O₂) comprising steps of:
a) providing a N₂O and O₂ premixture containing an initial content of N₂O comprised between 40% and 60% mol., the remainder being oxygen, said N₂O and O₂ premixture coming from a gas container,
b) diluting the N₂O and O₂ premixture with air delivered by a micro-blower, and
c) recovering a final mixture containing a final content of N₂O less than the initial content of N₂O in the N₂O and O₂ premixture.

2. Method according to claim 1, **characterised in that** the N₂O and O₂ premixture contains an initial content of N₂O comprised between 45% and 55% mol., the remainder being oxygen.

3. Method according to one of the preceding claims, **characterised in that** the N₂O and O₂ premixture contains 50% mol. of N₂O and 50% mol. of oxygen.

4. Method according to one of the preceding claims, **characterised in that** the N₂O and O₂ premixture is delivered by a gas cylinder.

5. Method according to claim 1, **characterised in that** the N₂O and O₂ premixture is delivered by a gas container equipped with a tap unit with an integrated gas expander.

6. Method according to claim 1, **characterised in that** in step b), the dilution of the N₂O and O₂ premixture with air is performed in a gas mixer comprising a mixing chamber, in particular a pressurised tank.

7. Method according to claim 1, **characterised in that** in step c), a final mixture containing a final content of N₂O comprised between 15% and 50% mol., preferably less than 40% mol. is recovered.

8. Method according to one of claims 1 or 7, **characterised in that** in step c), the final mixture containing at least N₂O, O₂ and N₂ is recovered.

9. Method according to claim 1, **characterised in that** it further comprises an additional step of controlling the content of oxygen (FiO₂) in the N₂O and O₂ premixture and/or in the final mixture.

10. System for providing gas comprising:
- an N₂O/O₂ premixture source (2) containing an initial content of N₂O comprised between 40% and 60% mol., the remainder being oxygen, the N₂O/O₂ premixture source (2) being in fluidic communication with a gas mixing chamber (1) by way of a N₂O/O₂ premixture conveyance line (4), the N₂O/O₂ premixture source (2) being a gas container,
- an air source (3) in fluidic communication with the gas mixing chamber (1) by way of an air conveyance line (5), the air source (3) being a micro-blower,
- a gas mixing chamber (1) producing a final gas mixture containing a final content of N₂O from the N₂O premixture created by the N₂O/O₂ premixture conveyance line (4) and air brought by the air conveyance line (5),
- a device for controlling air flow (6A) fitted on the air conveyance line (5) to control the air flow within the air conveyance line (5) and/or a device for controlling N₂O/O₂ premixture flow (6B) fitted on the N₂O/O₂ premixture conveyance line (4) to control the N₂O/O₂ premixture flow within the N₂O/O₂ premixture conveyance line (4),
- a man-machine interface (7) allowing an operator to enter several parameters comprising the initial content of N₂O in the N₂O/O₂ premixture, the content of N₂O in the final mixture and a desired final flow of gas (Q_{final}) of the final mixture produced in the gas mixing chamber (1), and
- calculation means (18) configured to act (19) on the device for controlling air flow (6A) and/or on the device for controlling N₂O/O₂ premixture flow (6B) so as to adjust the air flow within the air conveyance line (5) and/or the N₂O/O₂ premixture flow, according to the parameters entered by the operator on the man-machine interface (7),
and wherein said man-machine interface (7) is electrically connected to said calculation means (18).

11. System for providing gas according to claim 10, **characterised in that** the N₂O/O₂ premixture source (2) is a gas cylinder.

12. System for providing gas according to claim 10, **characterised in that** the device for controlling air flow (6A) and the device for controlling N₂O/O₂ premixture flow (6B) each comprising a solenoid valve, in particular a proportional solenoid valve.

13. System for providing gas according to claim 10, **characterised in that** the calculation means (18) comprise at least one microprocessor, in particular a microcontroller.

14. System for providing gas according to claim 10, **characterised in that** it further comprises a gas recovery line (8) in fluidic communication with the gas mixing chamber (1) to recover at least one portion of the final mixture produced in said gas mixing chamber (1) and convey the final mixture to the final flow (Q_{final}) entered by the operator on the man-machine interface (7).

15. System for providing gas according to claim 10, **characterised in that** the man-machine interface (7) comprises a touchscreen, preferably with a colour display.
